# EUROPÄISCHE PATENTSCHRIFT

(11) **EP 2 623 140 B1**
(45) Veröffentlichungstag und Bekanntmachung des Hinweises auf die Patenterteilung: **08.02.2017**
(21) Anmeldenummer: 13163421.4
(22) Anmeldetag: 07.09.2007
(51) Int. Cl.: A61M 1/36

(54) **Verfahren zur Unterstützung von Immuntherapien**
Method for supporting immune treatment
Procédé de soutien de immunothérapies

(30) Priorität: 07.09.2006 DE 102006042012
(43) Veröffentlichungstag der Anmeldung: 07.08.2013
(62) Teilanmeldung aus: 07802220.9
(73) Patentinhaber: Fresenius Medical Care Deutschland GmbH, 61352 Bad Homburg (DE)
(72) Erfinder: Hepper, Martin, 67435 Neustadt (DE); Leinenbach, Hans Peter, 66636 Tholey (DE); Nocken, Frank, 60320 Frankfurt (DE)
(74) Vertreter: Stolmár & Partner Patentanwälte PartG mbB

(56) Entgegenhaltungen:
- EP-A- 0 082 345
- US-B1- 6 406 861
- BORBERG ET AL: "Quo vadis haemapheresis", TRANSFUSION AND APHERESIS SCIENCE, ELSEVIER SCIENCE, LONDON, GB, Bd. 34, Nr. 1, Februar 2006 (2006-02), Seiten 51-73, XP005288266, ISSN: 1473-0502
- MONTGOMERY R A ET AL: "Plasmapheresis and intravenous immune globulin provides effective rescue therapy for refractory humoral rejection and allows kidneys to be successfully transplanted into cross-match-positive recipients.", TRANSPLANTATION 27 SEP 2000, Bd. 70, Nr. 6, 27. September 2000 (2000-09-27), Seiten 887-895, XP002457983, ISSN: 0041-1337
- KIEWE P ET AL: "PHASE I TRIAL OF THE TRIFUNCTIONAL ANTI-HER2 X ANTI-CD3 ANTIBODY ERTUMAXOMAB IN METASTATIC BREAST CANCER", CLINICAL CANCER RES, Bd. 12, Nr. 10, 15. Mai 2006 (2006-05-15), Seiten 3085-3091, XP008067039, ISSN: 0732-183X
- TAYLOR RONALD P ET AL: "Drug insight: the mechanism of action of rituximab in autoimmune disease--the immune complex decoy hypothesis.", NATURE CLINICAL PRACTICE. RHEUMATOLOGY FEB 2007, Bd. 3, Nr. 2, Februar 2007 (2007-02), Seiten 86-95, XP009091912, ISSN: 1745-8382
- BROWNING JEFFREY L: "B cells move to centre stage: novel opportunities for autoimmune disease treatment.", NATURE REVIEWS. DRUG DISCOVERY JUL 2006, Bd. 5, Nr. 7, Juli 2006 (2006-07), Seiten 564-576, XP002699784, ISSN: 1474-1776

## Beschreibung

Die vorliegende Erfindung betrifft die Verwendung spezifischer Liganden für körpereigene Antikörper oder für in Blutproben lösliche Krebs-assoziierte Antigene zur Herstellung einer Säule, die den Liganden daran gekoppelt aufweist, für die Behandlung von Krebs.

Neben der konventionellen Krebsbehandlung, die auf chirurgischen Maßnahmen, der Verwendung chemotherapeutischer Medikamente und/oder Bestrahlung basiert, hat sich die Immuntherapie mit Antikörpern etabliert. Gleichermaßen sind eine Vielzahl biotechnologischer Produkte, zu denen unter anderen auch die therapeutischen Antikörper zählen, zur Behandlung von Autoimmunerkrankungen als Therapieoption bereits anerkannt. Sogenannte HIV-neutralisierende Antikörper werden derzeit zur Behandlung von AIDS in klinischen Studien untersucht.

Die Zielstrukturen, die sogenannten Antigene, sind für die verschiedenen Erkrankungen sehr unterschiedlich und auch die Wirkweise der jeweiligen Antikörpertherapien kann in Abhängigkeit von diesen Zielstrukturen sehr verschieden sein. Oberflächenantigene, die als Zielstruktur zur Behandlung von malignen Erkrankungen dienen, sind u.a. überexprimierte, Krebs-assoziierte, membranständige Proteine (EGFR/HER2/VEGFR) oder auch Zell-spezifische Proteine (CD20/CD52). Die Anzahl der Antigen-positiven Zellen ist bei der Entwicklung von Lymphomen überaus groß, so dass eine Verminderung dieser Zellpopulation ein anerkanntes therapeutisches Ziel darstellt. Neben der Behandlung von Lymphomen eignet sich die zielgerichtete Depletion Antigen-positiver Zellen auch zur Behandlung von Autoimmunerkrankungen und gegebenenfalls zur Unterdrückung der Immunantwort wie z.B. bei der Transplantation. Antikörper oder auch Fusionsproteine, gerichtet gegen zelluläre Botenstoffe wie den löslichen Tumornekrosefaktor, sind wichtige Medikamente zur Behandlung von Autoimmunerkrankungen.

Die ersten modernen monoklonalen Antikörper (Immunglobuline meistens des IgGl-typus) waren murinen Ursprungs, d.h. sie wurden mittels Mäuse- oder Rattenhybridomzellinien hergestellt. Diese IgG1 Moleküle werden jedoch vom Körper des Menschen als fremd erkannt, so dass sie durch das menschliche Immunsystem neutralisiert werden. Aus diesem Grund wurden modernere, sogenannte chimäre Antikörper, bestehend aus murinen Anteilen und menschlichen Anteilen in der IgG Struktur, hergestellt. Die sogenannte Humanisierung, bis hin zur biotechnologisch optimierten Variante der kompletthumanen Antikörper, stellt den nächsten Schritt zur weiteren Minimierung der murinen Anteile dar. Chimäre, humanisierte und humane IgG1-Antikörper können über einen längeren Zeitraum, beispielsweise über Monate, während der Therapie eingesetzt werden. (Abdullah N., Cancer Immunther. 48, 517-524), (Adams GP, Weiner LM, Nature Biotechnology, 2005; 23(9): 1146 - 1157).

Außerdem werden Immuntherapien unter anderem mit Fcγ-Rezeptor-bindenden Agenzien durchgeführt. Fcγ-Rezeptoren (FCR) sind eine Familie von Rezeptoren, die spezifisch für die Fc-Teile von Immunoglobulin (IgG) sind. Diese Rezeptoren haben wichtige Aufgaben im normalen Immunsystem und dessen Widerstandsfähigkeit bei Infektionen. Somit sind IgG eine Klasse von Molekülen, die den Fcγ-Rezeptor binden.

Rezeptoren gibt es für jede Immunoglobulin-Klasse. Sie sind definiert durch die Klasse von Immunglobulin, an die sie binden. Beispielsweise bindet Fcγ-Rezeptor (FcγR) IgG, der Fcε-Rezeptor (FcεR) bindet IgE usw. Unter den FcγR-Rezeptoren werden drei Mitglieder von Unterfamilien unterschieden: FcγRI, der ein Rezeptor mit hoher Affinität für IgG ist, FcγRII, die Rezeptoren mit geringer Affinität für IgG sind, die jedoch gut an Aggregate von Immunkomplexen binden, und FcγRIII die Rezeptoren mit niedriger Affinität sind, die an Immunkomplexe binden.

Zwar sind alle diese Rezeptoren strukturell untereinander verwandt, sie haben jedoch verschiedene Aufgaben.

FcγR werden von den meisten hämatopoietischen Zellen exprimiert und spielen über die Bindung an IgG eine Schlüsselrolle bei der Homeostase des Immunsystems und dem Schutz gegen Infektionen. Insbesondere FcγRII ist ein Rezeptor mit niedriger Affinität für IgG, der im wesentlichen nur an IgG-Immunkomplexe bindet, und er wird auf einer Vielzahl von Zellstücken exprimiert, umfassend beispielsweise Monozyten, Makrophagen, Neutrophile, Eosinophile, Plättchen und B-Lymphozyten.

Fcγ-Rezeptoren sind in verschiedene Immun- und Entzündungsantworten einschließlich der Antikörperabhängigen, zellvermittelten Zytotoxizität (ADCC), involviert. Die Antikörper-vermittelte, zelluläre Zytotoxizität (ADCC) ist für die Wirkung biologischer Arzneimittel wie z.B. von poly- und monoklonalen Antikörpern, aber auch von Fusionsproteinen, verantwortlich. Die Effektivität der ADCC steht in direktem Zusammenhang mit der beschriebenen Interaktion der konstanten Region des Antikörpers, oder aber auch mit den Bindungseigenschaften des entsprechenden Proteins mit den Fcg-Rezeptoren. Für die ADCC scheint hierbei vor allem die Bindung an die aktivierenden Fcg I und III Rezeptoren zu sein; wohingegen die Bindung eines Arzneimittels vorwiegend an FcgII Rezeptoren die Immunantwort unterdrückt.

Ein kontinuierlicher Einsatz therapeutischer Proteine, wie z.B. von Antikörpern, bzw. von FcR bindenden Agenzien, insbesondere zur Behandlung von Krebs- und Autoimmunerkrankungen, Infektionskrankheiten und zur Unterdrückung von Transplantationsabstossungsreaktionen, wird dadurch eingeschränkt, dass diese Medikamente in hohen Dosen während einer kontinuierlichen Therapie appliziert werden müssen.

Bei der Anwendung von Antikörpern einer anderen, aber sogar auch humanisierter oder humaner Antikörper, werden diese Fremdproteine vom Immunsystem des Patienten erkannt und in Abhängigkeit von der jeweiligen Immunogenität durch die Entwicklung körpereigener (autologer) Antikörper (Humane anti-Spezies Antikörper) neutralisiert. Diese Immunantwort wird durch z.B. Humane Anti-Murine Antikörper (HAMA) im Falle muriner Antikörper, HARA im Falle von Antikörpern aus Kaninchen, (HAMA) im Falle muriner Antikörper bzw. durch Humane Anti-Humane Antikörper (HAHA) im Falle humanisierter oder humaner Antikörper vermittelt. Selbstverständlich induzieren auch mono- und polyklonale Antikörperprodukte, die aus anderen Spezies wie z.B. Ratten, Pferden, Ziegen, Schafen, Rinder oder auch Schweinen gewonnen werden, die Entwicklung von humanen Antikörpern gerichtet gegen die jeweilige Spezies, was oft zu starken Immunreaktionen und zu einem Verlust an Effizienz führen kann. Diese Form der körpereigenen Antikörper gegen Antikörper aus anderen Organismen werden zusammenfassend humane Anti-Spezies-Antikörper genannt.

Die Immunogenität der körperfremden, biotechnologischen Produkte beschränkt deren therapeutische Effektivität z.B. durch die Entwicklung der oben beschriebenen HAMA und HAHA Antwort des Immunsystems.

Die *in vivo* beobachtete relativ niedrige Wirksamkeit therapeutischer Antikörperpräparate steht in starkem Widerspruch zur oftmals sehr hohen Antikörper-vermittelten, zellulären Zytotoxizität (ADCC), die in vorherigen *in vitro* Untersuchungen nachgewiesen worden wurde. Dieses Phänomen tritt auch bei Kombinationstherapien herkömmlicher Arzneimitteltherapien, z.B. Chemotherapien, mit Immuntherapien auf. Wenn im folgenden von Arzneimitteln allgemein gesprochen wird, so sind damit herkömmliche, nicht immuntherapeutisch wirkende Arzneimittel gemeint, ganz besonders werden vorliegend darunter Chemotherapeutika und/oder Cytostatika verstanden. Eine mögliche Erklärung für dieses Phänomen stellt die beschriebene Inhibierung der therapeutischen Antikörper durch HAMA/HAHA dar (Preithner, S. et al. in Molecular Immunology, 43 (2006) 1183-1193.) Des weiteren stellen die natürlich vorkommenden IgG1 im Patientenblut Moleküle dar, die vergleichbar mit den therapeutischen Antikörpern um die entsprechenden Fc-Rezeptoren in Wettbewerb stehen, da auch diese IgG1 an die entsprechenden Rezeptoren binden und diese dadurch besetzen.

Es wurde erwartet, dass humanisierte oder humane Antikörper zu einer verbesserten Wirksamkeit und zu einem besseren Sicherheitsprofil führen würden. Wie vorstehend schon erwähnt, zeigen überraschenderweise auch fast alle humanisierten oder humanen Antikörper eine dramatisch verminderte Wirksamkeit in vivo, verglichen mit ihrer in vitro-Aktivität.

Daher ist es nötig, vielfach höhere Dosen von Antikörpern den Patienten zu verabreichen wodurch das Risiko unerwünschter Nebenwirkungen erhöht wird.

Im Fall einer gezielten Verwendung von klassischen Chemotherapien in Kombination mit Antikörper-basierten Immuntherapien, welche im Bereich der Onkologie ein akzeptierter Therapiestandard zur Behandlung verschiedener maligner Erkrankungen, wie z.B. bei Brustkrebs (Trastuzumab), Lymphomen (Rituximab) oder auch bei Darmkrebs (Cetuximab und Panitumumab) sind, wird, wie oben bereits angedeutet, *in vivo* ebenfalls nicht die erforderliche Wirksamkeit erzielt.

Die synergistische Effektivitätssteigerung der Kombinationstherapie wurde hierbei klinisch und präklinisch beschrieben. Neuere Erkenntnisse basierend auf *in vitro* Experimenten, belegen, dass die Interaktion mit die FcR-positiven Zellen, wie z.B. NK, DC und dergleichen, dem beobachteten Synergismus zu Grunde liegen. Es wurde nachgewiesen, dass es hierbei zum einen durch die Verwendung von Arzneimitteln, wie z.B. Paclitaxel (Miura D. et al., Journal of Clinical Oncology, 2007, Part I. Vol 25, No. 18S (June 20 Supplement) , Lenalidomiden und Pomalidomide (Bartlett J.B. et al., Journal of Clinical Oncology, 2007 ASCO Annual Meeting Proceedings Part I. Vol 25, No. 18S (June 20 Supplement)), aber auch Kinaseinhibitoren, wie z.B. Sorafinib (Hipp et al, Journal of Clinical Oncology, 2007 ASCO Annual Meeting Proceedings Part I. Vol 25, No. 18S (June 20 Supplement) einen direkten Einfluss auf die Homeostase der Antigen-präsentierenden Zellen aber auch auf verschiedene T-Zellpopulationen, wie z.B. cytotoxische T-Zellen (CTLs) haben. Für Sorafinib ist hierbei ein negativer Einfluss auf Antigen-präsentierende Zellen und die Entwicklung einer CTL-Antwort nachgewiesen worden, weshalb Kinaseinhibitoren wie Sunitib als geeigneter für eine Kombinationstherapie mit Immuntherapeutika erscheinen, ebenso verschiedene Her2 Kinaseinhibitoren wie z.B. Lapatinip oder auch Canertinib. In den oben zitierten Arbeiteten, wurde der synergistische Effekt auf die FcR-positiven Zellpopulationen zurückgeführt, die die ADCC vermitteln.

Klinisch ist bei den existierenden Kombinationstherapien leider zu verzeichnen, dass eine Mehrheit der behandelten Patienten Resistenzmechanismen gegen die verwendete Chemotherapie und gegen die entsprechende Antikörper basierte Immuntherapie entwickeln. Hierbei spielen unabhängig voneinander verschiedene Mechanismen eine Rolle. Für die oben aufgeführten Immuntherapien sind einige Target-abhängige aber auch Target-unabhängige Resistenzmechanismen beschrieben worden (Herceptin:Ritter C.A. et al.,Clinical Cancer Research 2007, 13(16):4909-4919; Valabrega G. et al., Annals of Oncology, 2007; Nahta R, et al., Natl Clin Pract Oncol 2006, 3:269-280; Esteva FJ et al., J Clin Oncol 2002, 20:1800-1808; Nagata Y et al., Cancer Cell 2004, 6:117-127; Scaltriti M et al., J Natl Cancer Inst 2007, 99:628-638; Rituximab: Van Meerten t et al., Clinical Cancer Research Vol. 12, 4027-4035, July 1, 2006).

R. A. Montgomery et al. beschreibt (Transplantation, Vol. 70, 887-895, September 27, 2000) ein Therapieverfahren um Abstoßungsreaktionen von Organtransplantaten zu verhindern. Dabei wird eine Plasmapherese (PP) mit intravenösem Gammaglubulin (IVIG) und Cytogam kombiniert. Durch eine Überdosis verabreichter Ig-Antikörper im Blut des Patienten kommt es zu einer Blockade von Fc-Rezeptoren und somit zu einer Immunsuppression. Eine Blockade der Fc-Rezeptoren ist im Sinne der Erfindung jedoch nicht gewünscht, sondern es sollen im Gegensatz dazu gerade die Fc-Rezeptoren von Antikörpern freigehalten werden, damit therapeutische Antikörper an diese Stellen binden und eine ADCC bewirken können. Eine Kombination von PP/IVIG und gegebenenfalls Cytogam soll also ausgeschlossen werden.

H. Borberg offenbart ein Verfahren zur Verringerung von IgG mittels einer anti-IgG Adsorptionssäule (H. Borberg, Transfusion and Apheresis Science, 34, 2006, 51-73) in Kombination mit einer IVIG Verabreichung. Die oben dargestellten Probleme einer Blockierung der Fc-Rezeptoren sind auch hier die Folge.

Das US 6,406,861 B1 offenbart ein Verfahren zur Verringerung von Virusantikörpern, insbesondere von Adenovirusantikörpern, durch extrakorporale Adsorption mit dem Ziel, die Effizienz einer Virusvektor-Therapie zu verbessern. Das US 6,406,861 B1 betrifft also keine Immuntherapie.

Es bestand daher die Aufgabe der vorliegenden Erfindung insbesondere darin, die Wirksamkeit von Fcγ-R bindenden Agenzien, insbesondere Antikörpern, zu verbessern, so dass die vorstehend geschilderten Nachteile vermieden werden können.

Gegenstand der vorliegenden Erfindung ist die Verwendung spezifischer Liganden für körpereigene Antikörper oder für in Blutproben lösliche Krebs-assoziierte Antigene zur Herstellung einer Säule, die den Liganden daran gekoppelt aufweist, für die Behandlung von Krebs, wobei die Behandlung die folgenden Schritte umfasst:
a) Bereitstellen einer Blutprobe eines Patienten;
b) Unterwerfen der Blutprobe einer Immunapherese unter Verwendung der Säule sowie des Verabreichens der so behandelten Blutprobe an den Patienten;
c) Verabreichen eines therapeutisch wirksamen Antikörpers an den Patienten,
wobei sich der therapeutisch wirksame Antikörper gegen Krebs-assoziierte Antigene richtet.

Vorzugsweise ist der Fcγ-Rezeptor bindende Wirkstoff im Rahmen der Erfindung ein therapeutischer Antikörper, besonders bevorzugt ein monoklonaler und/oder rekombinater Antikörper oder ein Wirkstoff mit FcR-bindenden Regionen, bestehend aus Antikörperfragmenten oder Peptiden gebunden an ein therapeutisches Agens.

Als zusätzlicher Schritt d) kann das Verabreichen eines Arzneimittels erfolgen. Dadurch erhält man eine Kombinationstherapie aus einer Arzneimitteltherapie mit einer Immuntherapie.

Vorzugsweise soll die Kombinationstherapie mit Immunglobulinen, deren Fragmenten oder auch Fusionsproteinen, die gezielt CD16 oder CD64 binden, erfolgen.

Weiterhin bevorzugt ist, dass die Immunglobuline, deren Fragmente oder Fusionsproteine neben der ADCC eine T-zellvermittelte Immunantwort induzieren.

Noch weiter bevorzugt ist, dass die Immunglobuline, deren Fragmente oder Fusionsproteine bispezifische Antikörper mit einem CD3 Bindungsarm beinhalten.

Ebenfalls bevorzugt ist eine Kombinationstherapie mit Immunglobulinen, deren Fragmenten oder auch Fusionsproteinen, bei der das Auftreten von Resistenzen durch die vorher genannten antitumoralen Zellantworten vermieden wird.

Bevorzugte Arzneimittel für die erfindungsgemäße Verwendung sind Cytostatika und Chemotherapeutika, insbesondere Paclitaxel, Lenalidomide, Pomalidomide, Epirubicin, 5FU und dessen Derivate, und Kinaseinhibitoren wie Sunitinib, Lapatinib, Canertinib. Weiterhin können auch Kombinationen aus verschiedenen Arzneimitteln wie CHOP, Cyclophosphamide, Doxorubicin, Vincristin Prednisolon (Steroid), Lenalidomiden/Dexamethason, Pomalidomid/Dexamethason und Paclitaxel/Carboplatin verwendet werden.

Bei der erfindungsgemäßen Verwendungen können Immunglobuline mit humanen, chimären, murinen, und hybrid Immunglobuline, deren Fragmente oder auch Fusionsproteine, die CD16/CD64 Bindungseigenschaften besitzen, eingesetzt werden. Weiterhin bevorzugt sind Immunglobuline, welche tumorassoziierte Antigene sind, Antigene, die mit Lymphomen oder Leukämie assoziiert sind, sowie Antigene, die mit Autoimmunkrankheiten assoziiert sind. Diese sind Her2/neu, EGFR, Epcam, VEGF, VEGFR,MUC-1,CA 125,CEA, MAGE, CD20, CD19, CD40, CD33, Kohlenstoffanhydrase IX, A3, Antigen spezifisch für A33 Antikörper, BrE3-Antigen, CD1, CDIa, CD4, CD5, CD8, CD14, CD15, CD16, CD21, CD22, CD23, CD25, CD30, CD37, CD38, CD40, CD40L CD45, CD 46, CD52, CD54, CD74, CD79a, CD80, CD126, CD138, CD154, B7, Ia, Ii, HM1.24, HLA-DR, NCA95, NCA90, HCG und Untereinheiten, CEA (CEACAM-5), CEACAM-6, CSAp, EGFR, EGP-I, EGP-2, Ba 733, Hypoxia induzierender Faktor (HIF), KC4-antigen, KS-I-antigen, KS1-4, Le-Y, Macrophagen inhibierender Faktor (MIF), MUC2, MUC3, MUC4, P1GF, ED-B Fibronectin, NCA 66a-d,PAM-4-Antigen, PSA, PSMA, RS5, SlOO, TAG-72, T101, TAG TRAIL-R1, TRAIL-R2, p53, Tenascin, IL-6, IL-8, Insulin Wachstumsfaktor-1 (IGF-I), Tn Antigen, Thomson-Friedenreich Antigene, Tumor Necrosis Antigene und Analoge davon und FcR-positive Zellen binden.

Im jeweiligen Schritt b) der oben genannten Verwendungen werden durch die Immunapherese der Blutprobe körpereigene Antikörper bzw. Fcγ-R bindende Agenzien entzogen.

Die Entfernung von humanen Anti-Spezies-Antikörpern und IgG-Antikörpern aus dem Patienten vor der Verabreichung des therapeutischen Antikörpers und im Falle einer Kombinationstherapie auch vor der Verabreichnung des Arzneistoffs, führt überraschenderweise dazu, dass die Wirksamkeit der Antikörpertherapie sowie auch der Kombinationstherapie um ein Vielfaches verbessert werden kann, und die Wirksamkeit *in vivo* nunmehr nahezu der erwarteten Wirksamkeit entspricht, wie sie *in vitro* bestimmt wurde. Die Dosierung therapeutischer Antikörper kann mindestens um das 5-fache, bevorzugt um das 10-fache in ganz besonders bevorzugten Ausführungsformen um das 20-fache vermindert werden, verglichen mit der Dosierung therapeutischer Antikörper bei herkömmlichen Verfahren.

Die Kombination aus Arzneimitteln mit Immuntherapien, die neben dem tumorassoziierten Antigen, gezielt FcR-positive Zellen binden, überwinden dadurch eine mögliche Resistenzentwicklungen, die durch die Antigene (Her2/EGFR/CD20), sekundäre intrazelluläre Signaltransduktionskaskaden (Apoptose oder PTEN-Verlust), oder andere Kinaseaktivitäten (HER3/PI3K/Akt) vermittelt sein könnten. Diese Immuntherapien rekrutieren gezielt Antigen-präsentierende Zellen, wodurch wiederum über costimulatorische (CD28/CD40) Signale T-Zellen aktiviert werden.

Ideale Kandidaten für eine Kombinationstherapie stellen humanisierte komplette und IgG-ähnliche bispezifische Antikörper (Asano et al.,2007, JBC) und trifunktionale bispezifische Antikörper, vorzugsweise Maus/Ratte IgG2a/IgG2b Chimäre, dar. Bei diesen Maus/Ratte IgG2a/IgG2b Chimären werden durch den zweiten CD-3-Bindungsarm sogar noch gezielt T-Zellen an die FcR I und III-positiven Zellen gebunden. Beide Rezeptoren zählen zu den ADCC vermittelnden Rezeptoren.

Neben den trifunktionalen Antikörpern, existieren bereits verschiedene andere Immuntherapien, die gezielt CD16 oder auch CD64 positive Zellen rekrutieren (Her2/CD16; CD30/CD16; CD19/CD64; CD15/CD64; Her2/CD64 u.s.w.) oder durch verbesserte FcR Bindungseigenschaften eine Verstärkung der ADCC ermöglichen. Auch solche Immuntherapien stellen geeignete Kandidaten für eine Kombinationstherapie dar, bei der potentielle Resistenzmechanismen umgangen und eine synergistische Therapie konzipiert werden kann.

Eine Kombinationstherapien mit humanisierten und IgG-ähnlichen bispezifschen Antikkörpern und/oder trifunktionalen Antiköpern und einer vorgeschalteten Immunadsorption bewirkt nicht nur eine stärkere Synergie bezüglich der ADCC und T-Zellvermittelten Cytotoxizität, sondern ermöglicht auch die Langzeitbehandlung der Kombinationstherapie mit den murinen Antikörpern. Die vorgeschaltete Immunadsorption neutralisiert auch die potentiell neutralisierenden Immunglobuline (HAMA, ADA), die sich gegen den therapeutischen Antikörper richten.

Durch die Entfernung der körpereigenen Antikörper fällt die Immunreaktion gegen die therapeutischen Antikörper wesentlich milder aus und die Wirksamkeit der therapeutischen Antikörper wird dadurch *in vivo* überraschenderweise gesteigert.

Mit Fcγ-Rezeptoren wechselwirkende Moleküle sind z. B. Immunoglobuline oder auch das pro-inflammatorische C-reaktive Protein (Das, T., FEBS Lett., 2004), die die zur Verfügung stehenden Fcγ-Rezeptoren besetzen.

Ein Beispiel ist die Antikörper-vermittelte zelluläre Zytotoxizität (ADCC) die für die Wirkung biologischer Arzneimittel, wie z.B. poly- und monoklonale Antikörper, aber auch von Fusionsproteinen verantwortlich. Die Wirksamkeit der ADCC steht in direktem Zusammenhang mit der Wechselwirkung der konstanten Region des Antikörpers aber auch mit den Bindungseigenschaften des entsprechenden Proteins mit den Fcγ-Rezeptoren. Für die ADCC scheint hierbei vor allem die Bindung an die aktivierenden FcγI- und III-Rezeptoren relevant zu sein. Die Bindung eines Arzneimittels vorwiegend an FcγII-Rezeptoren unterdrückt hingegen die Immunantwort. Beispielsweise gibt es bi-spezifische Antikörper, die gegen ein Target-Protein wie z.B. HER2 aber auch gleichzeitig gegen den Fcγ-Rezeptor I oder den Fcγ-Rezeptor III gerichtet sind.

Weitere bevorzugte Wirkstoffe, die Fcγ-Rezeptoren binden, sind typischerweise polyklonale und monoklonale Antikörper tierischen und humanen Ursprungs, rekombinante Antikörper, chimäre, primatisierte, humanisierte und humane monoklonale Antikörper, Antikörperdomänen und Fragmente davon, bi-, tri- und multispezifische Antikörper und Konstrukte von Antikörperfragmenten sowie Moleküle oder Wirkstoffe, die spezifisch Fcγ-Rezeptoren binden können, wie natürliche und rekombinante Proteine und Peptide, die mit dem Fcγ-Rezeptor wechselwirken, Fusionsproteine, die mit dem Fcγ-Rezeptoren wechselwirken, synthetische Peptide, die mit Fcγ-Rezeptoren wechselwirken und lösliche Fcγ-Rezeptoren. Ebenso werden darunter Behandlungen mit Wirkstoffen verstanden, die die Fcγ-Rezeptorenexpression beeinflussen, wie Behandlungen mit Zytokinen und Zytokinfusionsproteinen, Hormonen oder Derivaten, Steroiden, Glukokortikoiden und dopaminergen Substanzen.

Beispielsweise verdeutlicht die Entwicklung von Impfstrategien unter Ausnutzung des gezielten Ansteuerns von Fcg-Rezeptor-positiven Dendritischen Zellen, z.B. durch die Kopplung von DNA Impfstoffen an IgG Strukturen (Zhaoyang You et al., 2001, Cancer Research) die Bandbreite möglicher Anwendungen, die sich durch Fc-Rezeptoren und daran bindende Agenzien eröffnen.

Durch den Entzug der mit Fcγ-Rezeptoren wechselwirkenden Moleküle im Blut gibt es für einen anschließend verabreichten Fcγ-R bindenden Wirkstoff einen geringeren Wettbewerb um die Bindungsstellen, so dass die Fcγ-R bindenden Wirkstoffe nunmehr bevorzugt binden können.

Nach der Behandlung mit therapeutischen Antikörpern bzw. mit Fcγ-Rezeptoren bindenden Wirkstoffen, bzw. nach der Kombinationstherapie, können dem Patienten die körpereigenen Antikörper beispielsweise im Anschluss an die Verabreichung der Fc-R bindenden Agenzien ebenfalls rückgeführt werden.

Die zeitweise Entfernung von humanen Anti-Spezies Antikörpern, wie z.B. HAMA und HARA sowie von IgG-Antikörpern im Allgemeinen ist für den Patienten sicher und erhöht nicht das Risiko für Infektionen.

Erfindungsgemäß kann die Blutprobe menschliches Blut oder Blutplasma sein. Das Plasma kann dabei in einer vorgeschalteten Stufe z. B. durch Plasmafiltration oder Zellseparation durch Zentrifugieren des Blutes erhalten werden.

So kann beispielsweise das Blut des Patienten bzw. das Plasma auch in einem extrakorporalen Schritt über einen Adsorber geleitet werden, der Fcγ bindende Agenzien, insbesondere Antikörper, bindet. Das Blut oder Plasma kann dem Patienten anschließend rückgeführt werden.

Unter dem Begriff "spezifische Liganden" werden solche Liganden verstanden, die selektiv Antikörper aus dem Blut entfernen, jedoch nicht andere Bestandteile des Blutes.

Als spezifischer Ligand kann beispielsweise Protein A verwendet werden, bzw. Moleküle, die Fcγ-Rezeptoren in ihrer Wirkung äquivalent sind, Fragmente davon, künstliche Peptide, Proteine, usw. Weitere Beispiele sind nachstehend angegeben.

Die Matrix der erfindungsgemäß verwendeten Säule für die Immunapherese besteht dabei aus Sepharose oder Acrylverbindungen, wie sie z. B. in dem EP 222 146 B1 beschrieben sind.

Vor Aufbringung des Liganden wird die Matrix bevorzugt mit CN-Br oder ähnlich wirkenden Verbindungen aktiviert.

Bevorzugt werden als Liganden Protein A, Protein G, Peptide, oder AntiAntikörper verwendet. Weiterhin kommen als Liganden FcR-Rezeptoren, Fragmente davon, oder äquivalente natürliche oder synthetische Moleküle mit gleichwirkenden Bindungseigenschaften in Frage.

In weiteren bevorzugten Ausführungsformen der Erfindung wird die extrakorporale Entfernung von humanen Anti-Spezies Antikörpern und IgG-Antikörpern durch ein Kombinationssystem erreicht, das aus einer Vorrichtung für die Blutplasmaerzeugung und einer zweiten Vorrichtung, in der das Plasma über eine Adsorber-Kolonne geführt wird, besteht. Diese Adsorber-Säule arbeitet im Prinzip wie eine Chromatographie-Säule.

Die Adsorber-Säule besteht typischerweise aus einem biokompatiblem Plastikgehäuse und enthält 20 bis 1500 ml einer inerten Matrix, auf der spezifische Liganden mit einer Affinität beispielsweise gegenüber humanen Anti-Spezies-Antikörpern wie HAMA, HARA und menschlichem IgG1 immobilisiert sind.

Wenn das Plasma über die Adsorber-Kolonne geführt wird, werden die humanen Anti-Spezies-Antikörper und IgG1-Antikörper aus dem Patientenblut durch diese Liganden gebunden und daher aus dem Plasma eliminiert.

Derartige Systeme bzw. Materialien sind beispielsweise aus der EP 0 082 345 bekannt.

Die Adsorber-Säulen sind regenerierbar und können wiederholt verwendet werden.

In einem typischen Zweisäulensystem ist nur eine Säule in Gebrauch, wohingegen die zweite Säule automatisch regeneriert wird. Auf diese Weise kann die Geschwindigkeit für die Verminderung der abzureichernden Spezies während einer einzigen Behandlungssitzung um bis zu 60 % gesteigert werden.

Alternativ dazu können auch größere Säulen mit mehr selektiven Liganden und einer höheren Adsorptionskapazität als Einfach-Kolonnen verwendet werden.

Antikörper-basierte Therapien, die sich gegen lösliche und Zellmembran gebundene Krebs-assoziierte Antigene richten, befinden sich derzeit in der klinischen Evaluierung. Zu solchen Krebs-assoziierten Antigenen gehören unter anderem CH125, PSA, MUC1, MAGE-1, HER2, CEA, AFP, EpCAM. Der verwendete spezifische Ligand besitzt eine hohe spezifische Bindungsaffinität zu dem Krebs-assoziierten Antigen. Insbesondere spezifische Antikörper gegen Krebs-assoziierte Antigene können als Liganden verwendet werden.

Eine erfindungsgemäße spezifische Adsorption der löslichen Krebs-assoziierten Antigene führt dazu, dass die therapeutischen Antikörper oder auch Peptide an der Komplexierung mit frei löslichen Antigenen gehindert werden. Auf diese Weise wird die Wirkstoffkonzentration an der Zielstruktur, dem Antigen auf der Zelle, erhöht und somit die Wirksamkeit erhöht. Außerdem kann damit die Antikörper-vermittelte, zelluläre Zytotoxizität (ADCC) als primäre, gegen den Antigen-positiven Tumor gerichtete, und damit äußerst effiziente Immunantwort, ermöglicht werden. Weiter führt die spezifische Adsorption, beispielsweise von PSA, zu einer Verstärkung der Wirksamkeit löslicher T-Zell-Rezeptoren, die spezifisch an PSA binden.

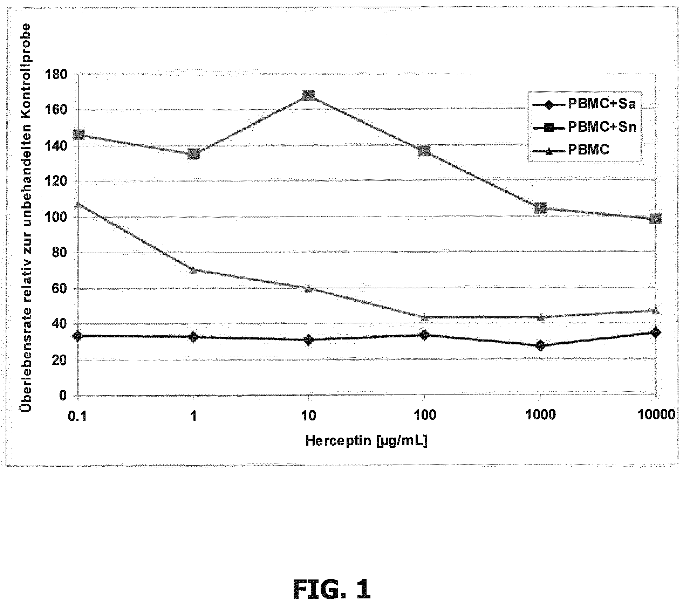

Die Erfindung wird weiter anhand eines nicht einschränkenden Beispiels unter Bezugnahme auf Figur 1 weiter erläutert.

### Beispiel 1:

### Aufhebung der Seruminhibition von Herceptin-vermittelter ADCC durch IgG-Adsorption:

### Experimenteller Aufbau:

1. Aussaat von Tumorzellen (Her-2/neu positive SK-OV-3 Ovarialzellen) über Nacht;
2. Isolation von PBMC (periphere mononukleare Blutzellen) aus dem buffy coat (Leukozytenfilm);
3. Co-Kultivierung von PBMC und Tumorzellen mit Herceptin unter Anwesenheit von IgG-depletierten (Sa) und nativem Serum (Sn);
4. Die Herceptin-Konzentrationen betragen 0,0001, 0,001, 0,01, 0,1, 1 und 10 µg/ml;
5. Die Inkubationszeit betrug 20 Stunden;
6. Die Messung der Zytotoxizität erfolgte mittels XTT;

Figur 1 zeigt, dass die Zytotoxizität der PBMC durch Zugabe von normalem, humanem Serum (Kurve PBMC + Sn) aufgehoben wird. Nach Zugabe von IgG-depletiertem Plasma (Kurve PBMC + Sa) bleibt die Zytotoxizität der PBMC erhalten. Im unteren Bereich der Herceptinkonzentration wird sie noch verstärkt.

## Patentansprüche

1. Verwendung spezifischer Liganden für körpereigene Antikörper oder für in Blutproben lösliche Krebs-assoziierte Antigene zur Herstellung einer Säule, die den Liganden daran gekoppelt aufweist, für die Behandlung von Krebs, wobei die Behandlung die Schritte umfasst des:
a) Bereitstellens einer Blutprobe eines Patienten;
b) Unterwerfens der Blutprobe einer Immunapherese unter Verwendung der Säule sowie des Verabreichens der so behandelten Blutprobe an den Patienten;
c) Verabreichens eines therapeutisch wirksamen Antikörpers an den Patienten,
wobei sich der therapeutisch wirksame Antikörper gegen Krebs-assoziierte Antigene richtet.

2. Verwendung nach Anspruch 1, **dadurch gekennzeichnet, dass** als Liganden Protein A, Protein G, Peptide und/oder AntiAntikörper verwendet werden.

3. Verwendung nach Anspruch 1, wobei die Krebs-assoziierten Antigene ausgewählt sind aus der Gruppe umfassend CH125, PSA, MUC1, MAGE-1, HER2, CEA, AFP und EpCAM.

4. Verwendung nach Anspruch 1 oder 3, **dadurch gekennzeichnet, dass** die Liganden spezifische Antikörper gegen Krebs-assoziierte Antigene sind.

5. Verwendung nach einem der Ansprüche 1 bis 4, **dadurch gekennzeichnet, dass** die Matrix der Säule aus Sepharose oder Acrylverbindungen besteht.

6. Verwendung nach einem der Ansprüche 1 bis 5, **dadurch gekennzeichnet, dass** die Matrix vor dem Aufbringen des Liganden mit CN-Br aktiviert wird.

7. Verwendung nach einem der Ansprüche 1 bis 6, **dadurch gekennzeichnet, dass** als zusätzlicher Schritt d) das Verabreichen eines Arzneimittels erfolgt.

8. Verwendung nach einem der Ansprüche 1 bis 7, **dadurch gekennzeichnet, dass** das Arzneimittel ein Chemotherapeutikum oder Cytostatikum ist.

9. Verwendung nach einem der Ansprüche 1 bis 8, **dadurch gekennzeichnet, dass** das Arzneimittel ausgewählt ist aus einer Gruppe umfassend Paclitaxel, Lenalidomid, Pomalidomid, Epirubicin, 5FU und dessen Derivaten, und Kinaseinhibitoren wie Sunitinib, Lapatinib, Canertinib, oder Kombinationen aus verschiedenen Arzneimitteln, ausgewählt aus der Gruppe umfassend CHOP, Cyclophosphamid, Doxorubicin, Vincristin, Prednisolon (Steroid), Lenalidomiden/Dexamethason, Pomalidomid/Dexamethason und Paclitaxel/Carboplatin.

10. Verwendung nach einem der Ansprüche 1 bis 9, **dadurch gekennzeichnet, dass** die in Schritt b) der Blutprobe entzogenen Antikörper dem Patienten im Anschluss an Schritt c) wieder verabreicht werden.

11. Verwendung nach einem der Ansprüche 1 bis 10, **dadurch gekennzeichnet, dass** die Blutprobe menschliches Blut oder Blutplasma ist.

12. Spezifische Liganden für körpereigene Antikörper oder für in Blutproben lösliche Krebs-assoziierte Antigene, wobei die Liganden an eine Säule gekoppelt sind, zur Verwendung in der Behandlung von Krebs, wobei die Behandlung die Schritte umfasst des:
a) Bereitstellens einer Blutprobe eines Patienten;
b) Unterwerfens der Blutprobe einer Immunapherese unter Verwendung der Säule sowie des Verabreichens der so behandelten Blutprobe an den Patienten;
c) Verabreichens eines therapeutisch wirksamen Antikörpers an den Patienten,
wobei sich der therapeutisch wirksame Antikörper gegen Krebs-assoziierte Antigene richtet.

## Claims

1. Use of specific ligands for endogenous antibodies or for in blood samples soluble cancer-associated antigens for producing a column having the ligand coupled thereto for the treatment of cancer, wherein the treatment comprises the steps of:
a) providing a blood sample of a patient;
b) subjecting the blood sample to an immunoapheresis using the column as well as administering the thus-treated blood sample to the patient;
c) administering a therapeutically effective antibody to the patient,
wherein the therapeutically effective antibody is directed against cancer-associated antigens.

2. Use according to claim 1, **characterized in that** protein A, protein G, peptides and/or anti-antibodies are used as ligands.

3. Use according to claim 1, wherein the cancer-associated antigens are selected from the group comprising CH125, PSA, MUC1, MAGE-1, HER2, CEA, AFP and EpCam.

4. Use according to claim 1 or 3, **characterised in that** the ligands are specific antibodies against cancer-associated antigens.

5. Use according to any of claims 1 to 4, **characterised in that** the matrix of the column consists of sepharose or acrylic compounds.

6. Use according to any of claims 1 to 5, **characterised in that** the matrix is activated with CN-Br prior to application of the ligand.

7. Use according to any of claims 1 to 6, **characterised in that** as an additional step of d) administering a drug is conducted.

8. Use according to any of claims 1 to 7, **characterised in that** the drug is a chemotherapeutic or a cytostatic.

9. Use according to any of claims 1 to 8, **characterised in that** the drug is selected from the group comprising paclitaxel, lenalidomide, pomalidomide, epirubicin, 5FU and its derivatives, kinase inhibitors such as sunitinib, lapatinib, canertinib, or combinations of different drugs selected from the group comprising CHOP, cyclophosphamide, doxorubicin, vincristine, prednisolone (steroide), lenalidomiden/dexamethasone, pomalidomide/dexamethasone and paclitaxel/carboplatin.

10. Use according to any of claims 1 to 9, **characterised in that** the antibodies extracted from the blood sample in step b) are administered back to the patient subsequent to step c).

11. Use according to any of claims 1 to 10, **characterised in that** the blood sample is human blood or blood plasma.

12. Specific ligands for endogenous antibodies or for in blood samples soluble cancer-associated antigens, wherein the ligands are coupled to a column, for use in the treatment of cancer, wherein the treatment comprises the steps of:
a) providing a blood sample of a patient;
b) subjecting the blood sample to an immunoapheresis using the column as well as administering the thus-treated blood sample to the patient;
c) administering a therapeutically effective antibody to the patient,
wherein the therapeutic effective antibody is directed against cancer-associated anti-gens.

## Revendications

1. Utilisation d'un ligand spécifique pour des anticorps endogènes ou pour des antigènes associés au cancer solubles dans des échantillons sanguins pour produire une colonne à laquelle le ligand est lié, pour le traitement du cancer, dans laquelle le traitement comprend les étapes de :
a) fournir un échantillon sanguin d'un patient;
b) soumettre l'échantillon sanguine à une immunophorèse ainsi qu'administrer l'échantillon sanguin ainsi traité au patient;
c) administrer un anticorps thérapeutiquement efficace au patient;
dans laquelle l'anticorps thérapeutiquement efficace se dirige contres des antigènes associés au cancer.

2. Utilisation selon la revendication 1, **caractérisée en ce que** protéine A, protéine G, des peptides et/ou des anti-anticorps sont utilisés comme ligand.

3. Utilisation selon la revendication 1, dans laquelle les antigènes associés au cancer sont sélectionnés du groupe comprenant CH125, PSA, MUC1, MAGE-1, HER2, CEA, AFP et EpCam.

4. Utilisation selon la revendication 1 ou 3, **caractérisée en ce que** les ligands sont des anticorps spécifiques contre des antigènes associés au cancer.

5. Utilisation selon l'une des revendications 1 à 4, **caractérisée en ce que** la matrice de la colonne consiste de sépharose ou des composés acryliques.

6. Utilisation selon l'une des revendications 1 à 5, **caractérisée en ce que** la matrice est activée par CN-Br avant l'application du ligand.

7. Utilisation selon l'une des revendications 1 à 6, **caractérisée en ce que** comme étape additionnelle l'étape de d) administrer un médicament est conduite.

8. Utilisation selon l'une des revendications 1 à 7, **caractérisée en ce que** le médicament est un chimiothérapeutique ou un cytostatique.

9. Utilisation selon l'une des revendications 1 à 8, **caractérisée en ce que** le médicament est sélectionné du groupe comprenant paclitaxel, lenalidomide, pomalidomide, epirubicin, 5FU et ses dérivés, des inhibiteurs de kinase comme sunitinib, lapatinib, canertinib, ou des combinaisons des médicaments différents sélectionnés du groupe comprenant CHOP, cyclophosphamide, doxorubicin, vincristine, prednisolone (steroide), lenalidomiden/dexamethasone, pomalidomide/dexamethasone et paclitaxel/carboplatin.

10. Utilisation selon l'une des revendications 1 à 9, **caractérisée en ce que** les anticorps extraits de l'échantillon sanguin dans l'étape b) sont administrés au patient suivant l'étape c).

11. Utilisation selon l'une des revendications 1 à 10, **caractérisée en ce que** l'échantillon sanguin est du sang humaine ou du plasma sanguin.

12. Ligands spécifiques pour des anticorps endogènes ou pour des antigènes associés au cancer solubles dans des échantillons sanguins, dans lesquelles les ligands sont liés à une colonne, pour l'utilisation dans le traitement du cancer, dans lesquelles le traitement comprend les étapes de :
a) fournir un échantillon sanguin d'un patient;
b) soumettre l'échantillon sanguine à une immunophorèse ainsi qu'administrer l'échantillon sanguin ainsi traité au patient;
c) administrer un anticorps thérapeutiquement efficace au patient;
dans laquelle l'anticorps thérapeutiquement efficace se dirige contres des antigènes associés au cancer.
